# EUROPEAN PATENT SPECIFICATION

(11) **EP 2 021 484 B1**
(45) Date of publication and mention of the grant of the patent: **10.03.2010**
(21) Application number: 07729769.5
(22) Date of filing: 31.05.2007
(51) Int. Cl.: C12N 15/867, C12N 15/861, C12N 15/863, C12N 15/869, C12N 5/10, A61K 48/00

(54) **EXPRESSION VECTORS COMPRISING THE HS1 PROMOTER OF THE VAV1 ONCOGENE AND USE THEREOF FOR THE PREPARATION OF PHARMACEUTICAL COMPOSITIONS INTENDED FOR SOMATIC GENE THERAPY**
EXPRESSIONSVEKTOREN MIT DEM HS1-PROMOTOR DES ONKOGENS VAV1 UND DEREN VERWENDUNG ZUR HERSTELLUNG VON PHARMAZEUTISCHEN ZUSAMMENSETZUNGEN FÜR DIE SOMATISCHE GENTHERAPIE
VECTEURS D'EXPRESSION COMPRENANT LE PROMOTEUR HS1 DE L'ONCOGÈNE VAV1 ET LEURS UTILISATIONS POUR LA PRÉPARATION DE COMPOSITIONS PHARMACEUTIQUES DESTINÉES À UNE THÉRAPIE GÉNIQUE SOMATIQUE

(30) Priority: 01.06.2006 ES 200601477
(43) Date of publication of application: 11.02.2009
(73) Proprietor: CENTRO DE INVESTIGACIONES ENERGETICAS MEDIOAMBIENTALES Y TECNOLOGICAS (C.I.E.M.A.T.), E-28040 Madrid (ES)
(72) Inventor: ALMARZA NOVOA, Elena, E-28040 Madrid (ES); GÜENECHEA AMURRIO, Guillermo, E-28040 Madrid (ES); SEGOVIA SANZ, José Carlos, E-28040 Madrid (ES); BUEREN RONCERO, Juan Antonio, E-28040 Madrid (ES); ALDEA GARCÍA, Montserrat, E-28040 Madrid (ES)
(74) Representative: Elzaburu Marquez, Alberto
(86) International application number: PCT/EP2007/055368
(87) International publication number: WO 2007/138104

(56) References cited:
- ALMARZA ELENA ET AL: "Regulatory elements of the vav gene drive transgene expression in hematopoietic stem cells from adult mice" EXPERIMENTAL HEMATOLOGY (NEW YORK), vol. 32, no. 4, April 2004 (2004-04), pages 360-364, XP002450208 ISSN: 0301-472X cited in the application
- OGILVY S ET AL: "Promoter elements of vav drive transgene expression in vivo throughout the hematopoietic compartment." BLOOD 15 SEP 1999, vol. 94, no. 6, 15 September 1999 (1999-09-15), pages 1855-1863, XP002450209 ISSN: 0006-4971 cited in the application
- OGILVY SARAH ET AL: "Transcriptional regulation of vav, a gene expressed throughout the hematopoietic compartment" BLOOD, vol. 91, no. 2, 15 January 1998 (1998-01-15), pages 419-430, XP002450210 ISSN: 0006-4971
- ALMARZA ELENA ET AL: "Characteristics of Lentiviral Vectors Harboring the Proximal Promoter of the vav Proto-oncogene: A Weak and Efficient Promoter for Gene Therapy." MOLECULAR THERAPY : THE JOURNAL OF THE AMERICAN SOCIETY OF GENE THERAPY AUG 2007, vol. 15, no. 8, August 2007 (2007-08), pages 1487-1494, XP009089238 ISSN: 1525-0016

## Description

### FIELD OF THE INVENTION

The present invention falls within the field of genetic engineering. Concretely the invention relates to the use of the HS1 promoter of the vav oncogene in the production of vectors selected from the groups of integrative vectors and of non-integrative, non-plasmid vectors, for use in the preparation of pharmaceutical compositions intended for somatic gene therapy. By using the HS1 promoter of the *vav* oncogene, it was possible to generate vectors in which the transgene is expressed at moderate, but stable, levels in various cell lines both *in vitro* and *in vivo.*

### BACKGROUND OF THE INVENTION

The development of gene therapy as a general method for the treatment of diseases will depend on a positive balance between the clinical benefit and the risk associated with this new therapeutic intervention. Although the efficacy of gene therapy for producing a clinical improvement in patients with hereditary disorders is already apparent¹⁻⁶, reservations have recently arisen concerning the safety of said therapy. Experimental studies have demonstrated the generation of leukaemias in mice associated with the activation of endogenous oncogenes by integrated proviruses.^{7,8} Moreover, similar results were observed in human patients, whose haematopoietic stem cells (HSCs) were transduced with gamma-retroviral vectors that contained viral promoters^{9,10}.

To date, it has been known that the regulatory sequences of the vav oncogene are effective for directing expression of transgenes in lympho-haematopoietic cells, including the self-renewing HSCs¹⁷. The regulatory sequences of the vav oncogene are constituted of five HS regions (hypersensitive to DNase), the HS1 region being the proximal promoter of the gene¹⁶. This HS1 region was capable of directing the specific expression of transgenes in stably transfected haematopoietic cell lines *in vitro*¹⁶, and it was demonstrated that the HS1 promoter region is sufficient for effecting expression in haematopoietic cell lines. In contrast to conditions *in vitro*, it has not been possible to conduct studies *in vivo* of expression controlled by HS1, since to produce transgenic mice it was necessary to insert other HS regions additional to HS1. On the other hand, the vectors used in the state of the art for carrying out cellular transfection were plasmid vectors^{5,16}.

In contrast to the initial studies of gene therapy in which the aim was to achieve high percentages of transduction with vectors that contained promoters of potent activity, the new trends highlight the convenience of controlling the number of proviral insertions in the cell's genome and the use of promoters that are incapable, if possible, of transactivating endogenous genes after stable insertion thereof in the cell's genome⁶. The advantage of limiting the influence of the promoter present in the vector on expression of genes near their site of integration has been reinforced by recent observations indicating that both the gamma-retroviral vectors and the lentiviral vectors have a high probability of insertion in regions of DNA near or within the genes¹¹⁻¹⁴.

### DESCRIPTION OF THE INVENTION

### Summary of the invention

In the present invention, integrative vectors have been developed that comprise the HS I promoter of the *vav* oncogene without the need for any other HS region, in particular without HS2 or HS5 having to be present in the gene construction that is claimed. The results obtained, which demonstrated moderate, stable expression of the marker transgene present in the lentiviral vector, indicated that vectors containing the vavHS1 promoter constitute a tool that is capable of controlling the stable, moderate expression of transgenes in mammalian cells both *in vitro* and *in vivo.* This limits the problems inherent in the vectors present in the state of the art which, since they include promoters that control a very high level of expression of the transgenes, facilitate the transactivation of other genes, including oncogenes, following integration in the genome of the host. Therefore, the use of vectors with promoters that control moderate expression of the transgene constitutes a safer tool for gene therapy.

### Description of the figures

**Fig. 1**
   **A.** The vavHS1-EGFP-LV lentiviral vector is a vector of 8393 bp in which the EGFP gene is under the control of the HS1-vav promoter.
   **B.** The CMV-EGFP-LV lentiviral vector is a vector of 7418 bp in which the EGFP gene is under the control of the promoter of the cytomegalovirus (CMV).
   **C.** The PGK-EGFP-LV lentiviral vector is a vector of 7384 bp in which the EGFP gene is under the control of the promoter of the phosphoglycerate kinase (PGK) gene.
   **D.** The SFFV-EGFP-LV lentiviral vector is a vector of 8029 bp in which the EGFP gene is under the control of the promoter of the "spleen focus forming virus" (SFFV).
**Fig. 2**
   shows a typical analysis of the activity of the CMV and vavHS1 promoters in transducing human B lymphoblasts (EBV-B cells) and HeLa epithelial cells with the CMV-EGFP-LV and vavHS1-EGFP-LV vectors in experimental conditions in which the efficacies of transduction corresponding to each vector in each line were similar. Each histogram shows the percentage of cells positive for EGFP as well as the mean fluorescence intensity (MFI) in the haematopoietic cell lines transduced with the CMV-EGFP-LV and vavHS1-EGFP-LV vectors.
**Fig. 3**
   shows the activity of the CMV and vavHS1 promoters in seven haematopoietic cell lines: CEM (human T lymphocytes), B (human B lymphoblasts), HL60 (human myeloid cells), WEHI (mouse myeloid cells), HEL (human erythroid cells), MEL (mouse erythroid cells) and FDCP1 (mouse haematopoietic stem cells). It shows the results 3-50 days after transduction of each cell line with two vectors containing the CMV and vavHS1 promoters. Similar multiplicities of infection (MOI) were used for infecting each line. The MOI represents the number of infectious viral particles relative to the number of cells to be infected. It is calculated by dividing the number of viral particles (ml added x infective units (i.u.) /ml) by the number of cells to be infected. For a higher value of MOI we can expect a larger number of copies of the provirus integrated per cell. According to published studies²⁰, the ratio of the percentage of cells expressing the transgene to the number of copies of the provirus integrated follows a Poisson distribution. A) shows the percentages of cells that are positive for EGFP, and B) shows the mean fluorescence intensity (MFI) in the haematopoietic cell lines transduced with the vectors CMV-EGFP-LV (black bars) and vavHS1-EGFP-LV (white bars).
**Fig. 4**
   shows the intensity of expression of the EGFP transgene (measured by the MFI) in two human myeloid haematopoietic lines, HL60 and K562 and two non-haematopoietic lines 293T (human epithelial) and 3T3 (mouse fibroblast), transduced with lentiviral vectors containing four different promoters: CMV promoter (black bars), vavHS1 promoter (white bars), phosphoglycerate kinase promoter (PGK; dot-shaded bars), and "spleen focus forming virus" promoter (SFFV; cross-hatched bars). In all cases the level of expression of the transgene was lowest in cells transduced with the vavHS1-EGFP-LV vector.
**Fig. 5**
   shows the activity of the vavHS1 promoter in mouse bone marrow cells in culture. It can be seen that there is stable expression of EGFP after transduction of stem cells not expressing differentiation markers (Lin⁻), with the vavHS1-EGFP-LV lentiviral vector at two different MOI: 2 (grey dots) and 0.2 (white dots), and cultivated *in vitro* for 45 days (abscissa).
**Fig. 6**
   shows the activity of the vavHS1 promoter in mouse haematopoietic cells *in vivo.* Days post-transplant are shown on the abscissa.
   **A.** The figure shows the levels of grafting of the donor haematopoietic cells (Ly5.2) in the recipients (Ly5.1), determined from the percentage of Ly5.2 cells in peripheral blood. The results obtained after transduction at a MOI of 2 (grey dots) and 0.2 (white dots) i.u./cell are shown.
   **B.** Data showing the percentage of transduced cells (EGFP⁺) in the Ly5.2 population of the donor at different times post-transplant. MOI: 2 (grey dots); MOI: 0.2 (white dots).
   **C.** The columns represent the percentage transduction (EGFP⁺) in cells of myeloid line (Gr/Mac⁺; black column), B lymphoid line (B220⁺; grey column) and T lymphoid line (CD3⁺; white column) at different times post-transplant. MOI: 2 (plain bars); MOI: 0.2 (dot-shaded bars).
   **D.** The columns represent the values of MFI of each of the transduced populations of a particular line at different times post-transplant. (Symbols as in C).
**Fig. 7**
   shows the activity of the vavHS1 promoter *in vivo* in human haematopoietic cells, after transduction of cells CD34⁺ from umbilical cord blood with the vavHS1-EGFP-LV lentivirus and transplant in NOD/SCID immunodeficient mice. Days post-transplant are shown on the abscissa.
   **A.** The figure shows the percentage of haematopoietic cells from the donor (cells positive to the CD45 marker) in the bone marrow of the transplanted immunodeficient mice. The CD34⁺ cells were transduced at a MOI of 3 (grey dots) and 0.3 (white dots) i.u./cell.
   **B.** Analysis of the percentage transduction of the donor haematopoietic cells in the transplanted immunodeficient mice. The results obtained after transduction at a MOI of 3 (grey dots) and 0.3 (white dots) i.u./ cell are shown.
   **C.** The columns represent the percentage of transduction (EGFP⁺ cells) in cells of immature line (CD34⁺; black column), myeloid line (CD33⁺; grey column), and B lymphoid line (CD19⁺; white column). MOI: 3 (plain bars); MOI: 0.3 (dot-shaded bars).
   **D.** The columns represent the MFI values in each of the transduced populations of a particular line. (Symbols as in C).

### Detailed description of the invention

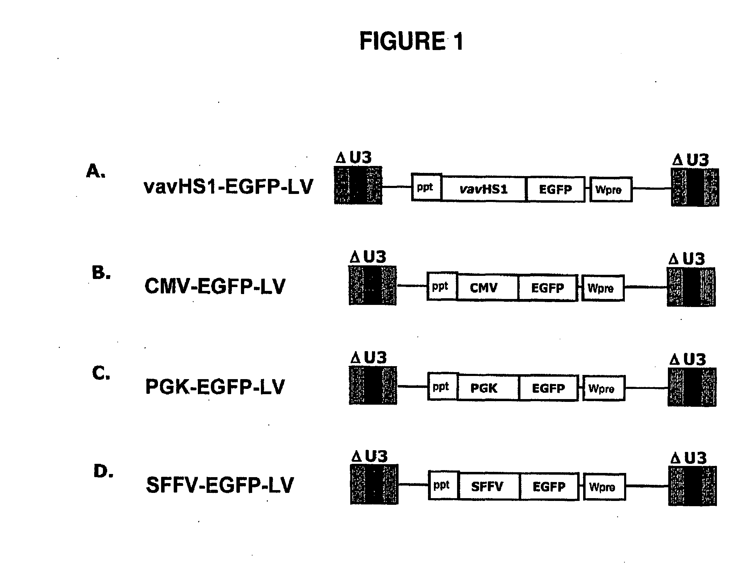

In the present invention, an integrative lentiviral vector has been developed, which comprises the vavHS1 promoter which controls expression of the EGFP marker gene (vavHS1-EGFP-LV) (Fig. 1). The results obtained showed that the vectors containing the vavHS1 promoter constitute a tool that is capable of controlling the stable, moderate expression of transgenes, in mammalian cells both *in vitro* and *in vivo,* limiting the problems inherent in the vectors used in the state of the art which, as they comprise promoters controlling strong expression of the transgenes, cause the transactivation of other genes, including oncogenes, after transduction of the host cell. Therefore, the use of vectors with promoters that control moderate expression of the transgene constitutes a safer tool for gene therapy.

As comparative controls, vectors were used that expressed the EGFP transgene under the control of the cytomegalovirus promoter (CMV-EGFP-LV), the phosphoglycerate kinase promoter (PGK-EGFP-LV), and the "spleen focus forming virus" promoter (SFFV-EGFP-LV) (Fig. 1).

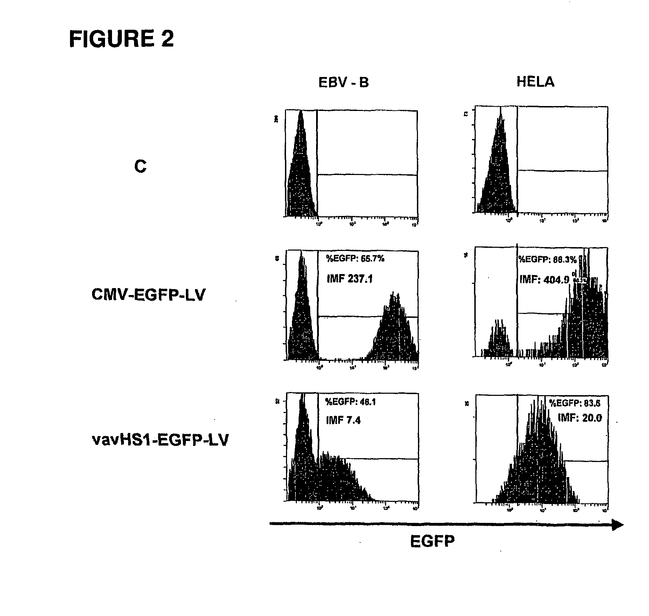

Fig. 2 shows typical histograms of the activity of the vavHS1 promoter compared with the MCV promoter (measured by the level of expression or MFI, of the EGFP transgene) in B lymphocytes and HeLa cells that had been transduced with the vavHS1-EGFP-LV and CMV-EGFP-LV vectors with comparable efficacy (measured from the percentage of cells positive to EGFP). The figure shows that the lentivirus carrying the vavHS1 promoter displays far lower activity, in comparison with the activity of the CMV-EGFP-LV lentivirus.

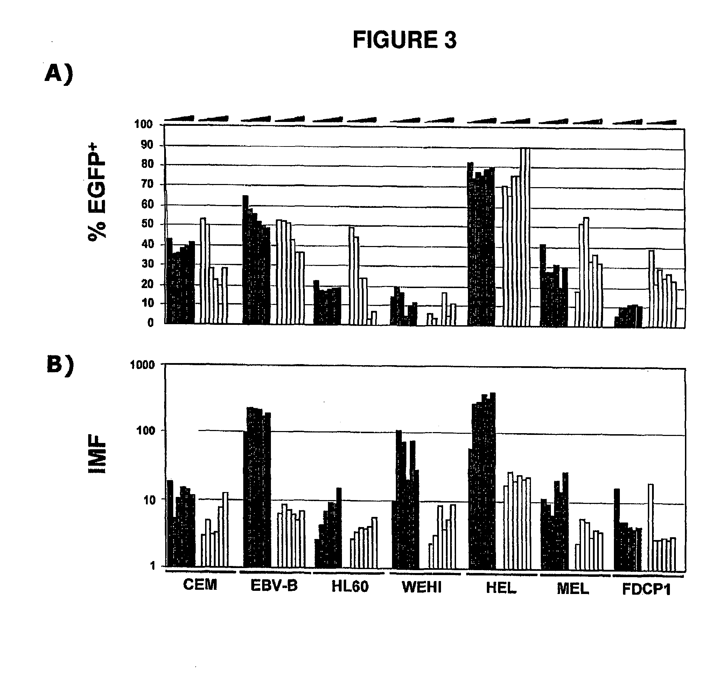

To confirm this observation a battery of haematopoietic cell lines was transduced with dilutions of supernatants containing vavHS1-EGFP-LV and CMV-EGFP-LVs. The cultures that displayed similar proportions of cells transduced by vavHS1-EGFP-LVs and CMV-EGFP-LVs were used for investigating the level and stability of expression of vavHS1-EGFP-LVs in comparison with CMV-EGFP-LVs. Fig. 3A shows the proportion of EGFP⁺ cells after culture for 3-50 days. As expected, expression of EGFP was evident in all the haematopoietic cells tested (B lymphocytes, T lymphocytes, myeloid cells and erythroid cells), after transduction both with vavHS1-EGFP-LVs and with CMV-EGFP-LVs. The figure also shows that there is no evidence of loss of expression of EGFP in the cells transduced with any of these vectors, which demonstrates that said expression is stable. For investigating the level of expression of EGFP in haematopoietic cells transduced with vavHS1-EGFP-LVs and CMV-EGFP-LVs, we determined the MFI of the EGFP in the respective cell lines (Fig. 3B). As shown in that figure, the MFI of EGFP observed in the cells transduced with vavHS1-EGFP-LVs was significantly less than that observed for cells transduced with CMV-EGFP-LVs. Another significant observation is that the activity of the promoter (measured by the MFI) was very similar in the different haematopoietic lines when these were transduced with the vavHS1-EGFP-LV vector (coefficient of variation; C.V.: 13.0), in contrast to the results obtained in cells transduced with CMV-EGFP-LV, where the values of MFI were highly dependent on the cell line (C.V.: 345.4).

Based on these analyses, it was concluded that expression of EGFP in haematopoietic cells transduced with vavHS1-EGFP-LVs was weaker and more uniform (regardless of the cell line transduced), than that observed in cells transduced with CMV-EGFP-LVs, where expression was more potent and heterogeneous; i.e. dependent on the cell line transduced. In no case were problems connected with the stability of expression of the transgene inferred from these studies.

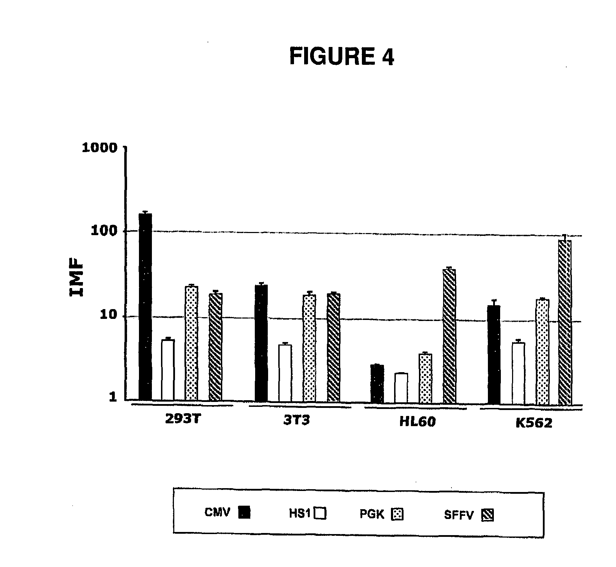

The observations shown in Fig. 3 regarding moderate activity of the vavHS1 promoter were confirmed in new tests, using, as controls, other lentiviral vectors in which the marker protein (EGFP) was controlled by the PGK, SFFV, and also CMV promoters (Fig. 4). The conclusions concerning the characteristics of the vavHS1 promoter were not only valid for all these promoters, but were also confirmed using both haematopoietic (HL60 and K562), and non-haematopoietic lines (293T and 3T3). In contrast to what was observed in the state of the art, which did not show any activity of the vavHS1 promoter in 3T3 cells transduced with HS1-LacZ gene constructions¹⁶, the results obtained with the vavHS1-EGFP-LV lentiviral vector show expression of EGFP in 3T3 cells.

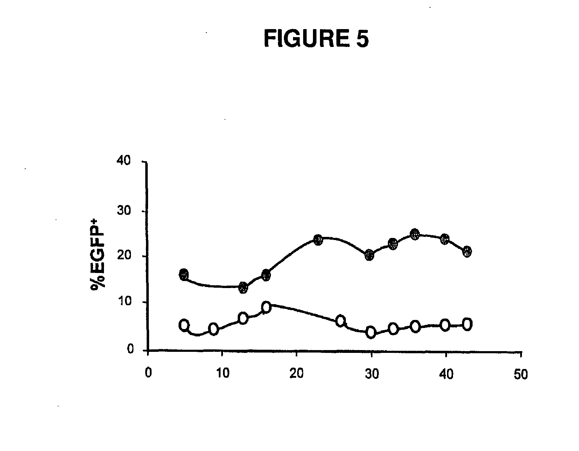

For investigating the pattern of expression of vavHS1-EGFP-LVs in mouse haematopoietic stem cells, bone marrow cells negative for lineage markers, and derived from B6D2F1 animals (Ly5.2) were pre-stimulated for 20 hours. Then they were transduced at two different MOI (2 and 0.2 i.u./cell) and were transplanted in P3D2F1 animals (Ly5.1). Additionally, an aliquot of the cells transduced was kept in culture for 45 days, for analysing the expression of EGFP in these cells *in vitro.* As was to be expected, the proportion of EGFP⁺ cells was dependent on the MOI (20% and 5%, respectively, at high and low MOI; Fig. 5), and was also stable during the period of observation (45 days of culture *in vitro*).

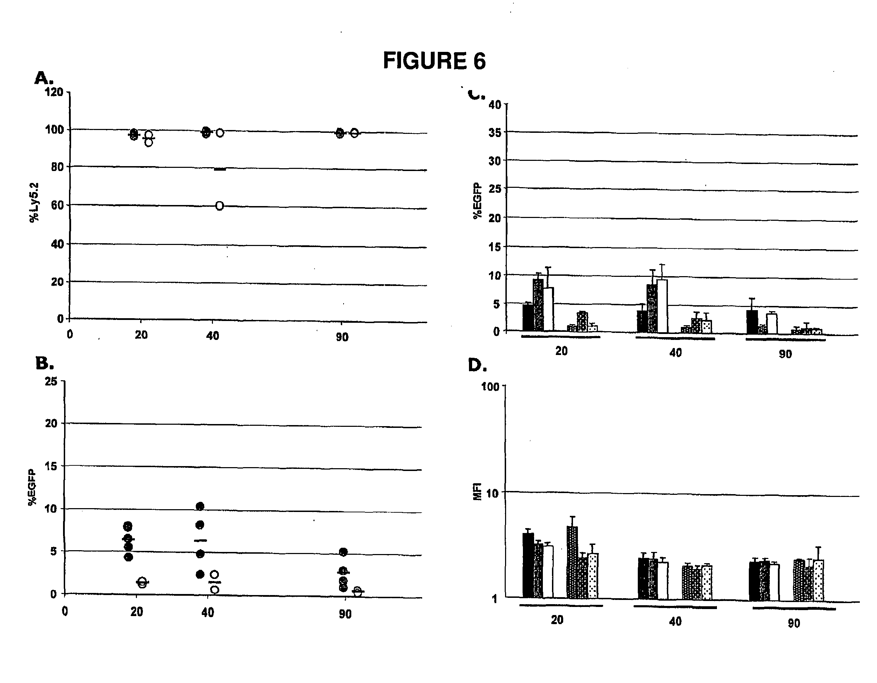

For investigating the expression of EGFP in haematopoietic cells *in vivo,* samples of peripheral blood of mice transplanted with the transduced samples were analysed at different times post-transplant (Fig. 6). As can be deduced from the proportion of Ly 5.2 cells in the peripheral blood of these animals, it is concluded that there is a high level of exogenous reconstitution (the majority of the animals showed values of Ly5.2 close to 100%; Fig. 6A). When the proportion of cells transduced in the donor cells was investigated (Fig. 6B), a proportion of EGFP⁺ cells dependent on the MOI (10% and 2% at a high and low MOI, respectively), and stable, was observed, as was to be expected.

For investigating the activity of vavHS1-EGFP-LVs in the different haematopoietic lineages *in vivo,* the MFI of EGFP in myeloid cells (Gr/Mac⁺), B lymphoid cells (B220⁺) and T lymphoid cells (CD3⁺; Fig. 6C) was analysed. Consistently with the results obtained in the cell lines (Fig. 3), expression of the transgene remained stable in all the lineages analysed (Fig. 6C). In addition, very similar MFI values were obtained in the myeloid, T lymphoid and B lymphoid lineages of the mouse (Fig. 6D).

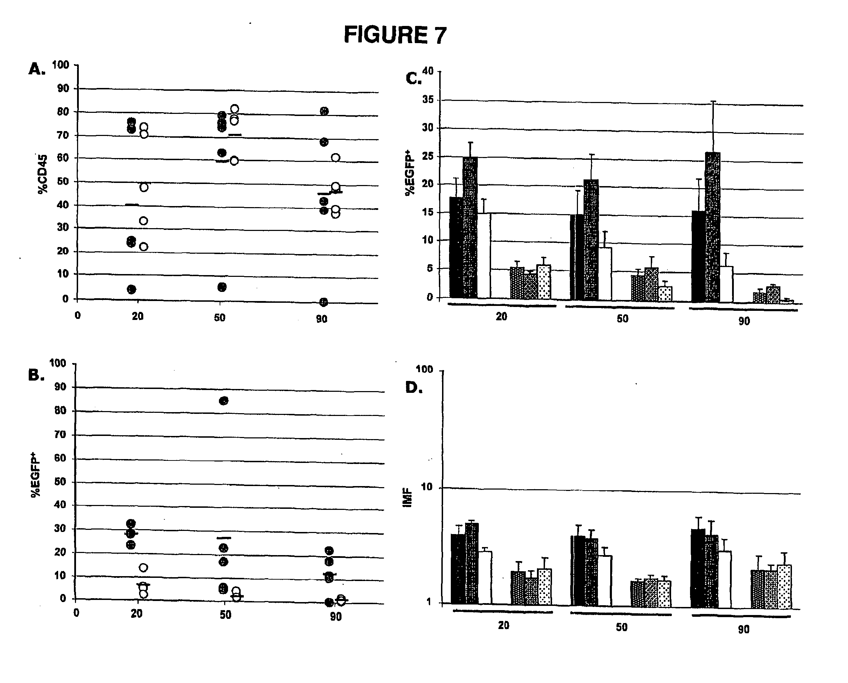

In order to confirm the earlier observations on the activity of the vavHS1 promoter in human primary haematopoietic cells, haematopoietic progenitor cells, CD34⁺, obtained from human umbilical cord blood were transduced with the vavHS1-EGFP-LV vector at two different MOI (3 and 0.3 i.u./cell). Selection of the population of CD34⁺ cells enables us to enrich the population with human HSCs. The CD34⁺ cells that had undergone the process of transduction were transplanted in irradiated NOD/SCID immunodeficient mice. Following transplant, expression of the EGFP marker gene in the human haematopoietic cells was investigated (Fig. 7).

As can be deduced from the proportion of cells positive to the CD45 marker (expression of which is specific to human haematopoietic cells), the majority of the animals showed high levels of grafting with human haematopoietic cells (70% on Day 50 post-transplant; Fig. 7A). When the percentage of EGFP⁺ cells in the population of human cells (CD45⁺) was analysed, stable expression of the transgene, dependent on the MOI, was found (Fig. 7B), similar to what was observed following transplant of mouse cells (Fig. 6B).

Analysis of EGFP in human haematopoietic stem cells (CD34+), in myeloid cells (CD33+) and in B lymphoid cells (CD19⁺), demonstrated that the percentage of EGFP⁺ cells remained stable over time, in each cell type (Fig. 7C).

Consistently with the observations made in previous experiments, when the level of expression of the transgene in the different cell types was analysed, very similar MFI values were observed in all the cell lines tested (Fig. 7D). As expected, the lowest values of MFI corresponded to the samples that had been transduced at a low MOI.

Thus, a first aspect of the present invention relates to the use of the vavHS1 promoter of the *vav* oncogene, characterized by the sequence SEQ ID NO: 1, in the production of vectors selected from the groups of integrative vectors and of non-integrative, non-plasmid vectors, for use in the preparation of pharmaceutical compositions intended for somatic gene therapy.

In a preferred embodiment, the present invention relates to the use of the vavHS1 promoter of the *vav* oncogene, characterized by the sequence SEQ ID NO: 1, in the production of vectors selected from the groups of integrative vectors and of non-integrative, non-plasmid vectors, for use in the preparation of pharmaceutical compositions intended for somatic gene therapy where said therapy is carried out "*in vivo*", in mammals.

In another preferred embodiment, the present invention relates to the use of the vavHS1 promoter of the *vav* oncogene, characterized by the sequence SEQ ID NO: 1, in the production of vectors selected from the groups of integrative vectors and of non-integrative, non-plasmid vectors, for use in the preparation of pharmaceutical compositions intended for somatic gene therapy where said therapy is carried out "*in vitro*" in cell cultures.

In another preferred embodiment, the present invention relates to the use of the vavHS1 promoter of the *vav* oncogene, characterized by the sequence SEQ ID NO: 1, in the production of vectors selected from the groups of integrative vectors and of non-integrative, non-plasmid vectors, for use in the preparation of pharmaceutical compositions intended for somatic gene therapy, where said therapy is carried out "*in vivo*" in mammals or *"in vitro*" in cell cultures and the vector is selected from the group of integrative vectors comprising gamma-retroviruses, lentiviruses, foamy viruses and adeno-associated viruses or from the group of non-integrative vectors comprising adenovirus, herpesvirus, poxvirus, vaccinia and liposomes and derivatives.

In a second aspect, the present invention relates to the use of the vectors of the invention for the preparation of Pharmaceutical compositions intended for use in somatic gene therapy.

In a third aspect, the present invention relates to the cells transduced by any of the vectors of the invention.

In a fourth aspect, the invention relates to the use of said cells, transduced by any of the vectors of the invention, for the preparation of pharmaceutical compositions intended for use in somatic gene therapy.

The final aspect of the present invention relates to pharmaceutical compositions comprising any of the vectors of the invention and pharmaceutically acceptable vehicles or comprising cells transduced by any of the vectors of the invention and pharmaceutically acceptable vehicles.

The examples will now be presented. The detailed description of the embodiments, the examples and the appended diagrams are given by way of illustration and are not intended to limit the present invention.

### EXAMPLES OF APPLICATION OF THE INVENTION

### Example 1. Synthesis of vectors.

Third-generation, self-inactivating lentiviral vectors were used in the present example. Concretely, the following were used: the pRRLsin18.pptCMVeGFPWpre transfer vector (EGFP, enhanced green fluorescent protein; Clontech), the pMDLg-pRRE and pRSV-REV packaging vectors and the pMD2-VSVG envelope vector^{18,19}. To generate the vavHS1 lentiviral vector (pRRLsin18.pptHS1CopGreenWpre), the enhanced green fluorescent protein (Cop-Green, EVROGEN) was amplified by PCR and cloned in the HS21/45*vav* plasmid^{15,16}. The resultant plasmid *vav* HS21/45-EGFP was directed to obtain a consistent fragment in the HS1 region of the *vav* promoter and the CopGreen transgene. This fragment was cloned in the pRRLsin18.pptCMVeGFPWpre vector^{18,19}, thus replacing the CMV promoter and the EGFP protein with the HS1 promoter and the CopGreen protein (Fig. 1). Both the fluorescent green protein EGFP from Clontech and the CopGreen protein from Evrogen have the same characteristics with respect to MFI when expressed under the same promoter in an equal number of copies (data not shown). Other transfer vectors used were pRRLsin18.ppthPGKeGFPWpre and pCLI.SFFVeGFP (Fig. 1).

### Example 2. Production of supernatant of Lentivirus.

The supernatants containing the lentiviral vectors were produced by transient cotransfection of the pRRLsin18.pptCMVeGFPWpre, pRRLsin18.ppthPGKeGFPWpre, pCL1.SFFVeGFP or pRRLsin18.pptHS1vavCopGreenWpre transfer vectors (9 µg), the pMDLg-pRRE (5.85 µg) and pRSV-REV (2.25 µg) packaging vectors and the pMD2-VSVG envelope vector (3.15 µg) in 10 cm dishes with 293T cells (ATCC) at 60-70% confluence. For transient transduction with these vectors, the protocol as described previously was carried out (Vigna E., Molecular Therapy, 11:763-775, 2005; Dull T. et al., Journal of Virology, Nov 1998, p. 8463-8471). For transient transfection with the transfer vector, the Polyfect transfection reagent (Qiagen) was used, following the manufacturer's instructions. In both cases sodium butyrate 1 mM (Sigma) was added to promote viral production. The supernatants that were recovered 24 and 48 hours after transduction were filtered through 0.45 µm filters and were concentrated by ultracentrifugation at 18000 rpm and 4°C for 90 minutes.

The titre of the lentiviral supernatants was determined in HeLa cells by FACS analysis and expression of GFP in an EPICS XL flow cytometer (Coulter Electronics, Hialeah, FL, USA). Titres close to 10⁷ v/ml were obtained with both vectors.

### Example 3. Cell lines transduced.

The human non-haematopoietic cell lines used were HeLa and 293T epithelial cells, and the human haematopoietic cell lines used were the CEM T lymphocyte cell line, the HL60 myeloid cell line and the HEL erythroid cell line. Lymphoblasts transduced with Epstein-Barr virus (EBV) from healthy donors were also used as B lymphocyte line.

The murine non-haematopoietic cell lines used were NIH 3T3 murine fibroblasts, from ATCC (Rockville, MD, USA). The murine haematopoietic cell lines used were murine myeloid cells WEHI, murine erythroid cells MEL, and murine haematopoietic stem cells FDCP1 from ATCC (ATCC number CRL-12103, Rockville, MD, USA).

The HeLa, 293T, NIH 3T3, and MEL cells were grown in Dulbecco's Modified Eagle's Medium (DMEM, Gibco Laboratories, Grand Island, NY, USA) supplemented with 10% fetal bovine serum (FBS; Cambrex), antibiotics (100 U/ml penicillin and 50 µg/ml streptomycin) and L-glutamine 2 mM. The FDC-P1 cells were grown in modified DMEM (ATCC number 30-2002) supplemented with 10% FBS, antibiotics (100 U/ml penicillin and 50 µg/ml streptomycin) and 2.5% IL-3 (supplement for culture of interleukin-3, BD Biosciences). The CEM, HEL, WEHI cells and the B lymphoblasts were grown in RPMI 1640 (Roswell Park Memorial Institute medium, GIBCO-BRL Laboratories), supplemented with 10% FBS, antibiotics and L-glutamine 2 mM. The HL60 cells were grown in Iscove's modified Dulbecco's medium (IMDM; GIBCO-BRL, Grand Island, NY), supplemented with 20% FBS and antibiotics (100 U/ml penicillin and 50 µg/ml streptomycin).

### Example 4. Murine models used.

NOD/LtSz-scid/scid (NOD/SCID) mice (deficient in Fc receptors, the complement function and the B and T lymphocyte and natural killer function) were used as recipients of the human haematopoietic cells. The mice were purchased from The Jackson Laboratory (Bar Harbor, ME). All the animals were handled in sterile conditions and were kept in microisolator cages. Prior to transplant, at age 6 to 8 weeks the mice received whole-body irradiation with 2.5 to 3.0 Gy of X-rays (the dose rate was 1.03 Gy/min using MG-324 X-ray equipment from Philips, Hamburg, Germany, at 300 kV, 10 mA).

The B6D2F1 and P3D2F1 colony founders were obtained from the Jackson Laboratories and were crossed in the animal house of the Energy, Environment and Technology Research Centre (registration number 28079-21 A). The mice were kept in conditions of high quality (high-efficiency particulate air filtered [HEPA], temperature controlled at 22°C, 12-hour cycles of light and darkness, and food and water irradiated with ultraviolet light *ad libitum*) and underwent routine examinations to detect possible pathogens. Prior to transplant, at age 10-12 weeks the mice were exposed to lethal irradiation with two doses of 4.75 Gy, 24 hours apart²¹, with MG-324 X-ray equipment (300 kV, 12.8 mA, Philips, Hamburg, Germany).

All the experimental procedures were carried out in accordance with Spanish and European regulations (RD 223/88 and OM 13-10-89 of the Spanish Ministry of Agriculture, Fisheries and Food, for the protection and use of animals in scientific research; and European Convention ETS-123, for the use and protection of vertebrate animals used in experiments and for other scientific purposes).

### Example 5. Transduction of cells.

The cell lines were transduced by adding different volumes of the concentrated supernatants of *Lentivirus* to a known number of cells depending on the multiplicity of infection (MOI) used. Different MOI were used in each cell line with the intention of keeping the efficiency of transduction below 40% and, therefore, the number of integrated copies close to one copy per cell. The cells were centrifuged for 1.5 h at 2500 rpm and 37°C, 2 and 24 hours after infection, to promote the efficiency of transduction.
The cells that were positive for EGFP were analysed by flow cytometry in an EPICS XL flow cytometer (Coulter Electronics, Hialeah, FL).

### Example 6. Analysis by flow cytometry.

Characterization of the recipient mice was carried out with peripheral blood cells and bone marrow cells.

Prior to analysis by flow cytometry, the erythrocytes were lysed for 10 minutes at room temperature in a lysis solution with ammonium chloride (NH₄Cl 0.155mM + KHCO₃ 0.01mM + EDTA [ethylenediamine tetraacetic acid]) 10⁻⁴ mM and were washed with PBA (phosphate-buffered saline solution [PBS] 1x + bovine serum albumin [BSA] 0.1% + NaN₃ 0.02%).

The peripheral blood cells from the P3D2F1 mice were stained with Ly5.2-biotinylated monoclonal antibodies (MoAbs), for identification of reconstitution of the animals owing to the donor and with Ly5.1-PE (for recognizing endogenous cells) and B220-biotinylated, Mac-biotinylated, Gr1-biotinylated, and mCD3-biotinylated (all from Pharmingen, Palo Alto, CA) for recognizing, respectively, B lymphocytes, monocytes, granulocytes and T lymphocytes. The cells were then stained with streptavidin-tricolor (Caltag, Burlingame, CA) for 30 minutes at 4°C and were washed again with PBA.

NOD/SCID mouse bone marrow cells were stained with anti-human CD45-PECy5 monoclonal antibodies (Clone J33, Immunotech, Marseilles, France), for identifying the human reconstitution of these animals, and in combination with anti-human CD34-PE (Clone 8G12; Becton Dickinson Immunocytometry, San Jose, CA), anti-human CD33-PE (P67.6; Becton Dickinson), or anti-human CD19-PE (J4.119; Immunotech) for recognizing human undifferentiated cells, myeloid cells or B lymphoid cells, respectively.

Finally, the cells were washed, resuspended in PBA with 2 µg/mL of propidium iodide (PI), and analysed in an EPICS XL flow cytometer (Coulter Electronics, Hialeah, FL). A minimum number of 10⁴-10⁵ viable cells was obtained.
The analysis was performed without connecting to the Internet with the free software package WinMDI (kindly supplied by Dr. J. Trotter, The Scripps Research Institute, La Jolla, CA).

### Example 7. Mouse samples and depletion of lineages.

Bone marrow samples were obtained from femurs of dead B6D2F1 animals (Ly5.2). The samples were stained first with a cocktail of differentiation markers (Lin) containing the B220-biotinylated, MAC-biotinylated, GR1-biotinylated and CD3-biotinylated MoAbs and were washed with phosphate-buffered saline solution (PBS) containing 5% bovine serum albumin (BSA) (Fraction V; Sigma, San Luis, MO) and 5 mM EDTA. Then the cells were stained with anti-biotin microspheres (Miltenyi Biotec., Germany) and were submitted to magnetic separation following the manufacturer's instructions.

### Example 8. Transduction, culture and transplant of mouse cells that are negative for expression of lineage markers

The bone marrow cells obtained, which are negative with respect to the lineages analysed, were then pre-stimulated for 20 hours in IMDM supplemented with 20% FBS, antibiotics (100 U/ml penicillin and 50 µg/ml streptomycin), L-glutamine 2 mM, and mSCF and IL11 to a final concentration of 100 ng/ml. These cells, which are negative to expression of the lineages analysed, were then transduced at two MOI (2 and 0.2 i.u./cell) with the lentivirus supernatants both with CMV and with HS1 for 24 hours. During this time the cells were centrifuged, 2 and 20 hours after transduction, for 1.5 h at 2500 rpm and 37°C. Then the cells were washed, and 3 to 7x10⁵ cells per mouse were transplanted in lethally irradiated P3D2F1 recipient mice (Ly5.1). Peripheral blood cells from these animals were analysed periodically by flow cytometry.

A small aliquot of the cells was cultured in IMDM supplemented with 20% FBS, antibiotics (100 U/ml penicillin and 50 µg/ml streptomycin), L-glutamine 2 mM, and mSCF and IL11 to a final concentration of 100 ng/ml. These cells were kept in culture for 45 days at 37°C and with 5% CO₂.

### Example 9. Human samples and selection of cells positive for CD34.

Cells were obtained from umbilical cord blood (CB) after a normal birth at full term, and with the informed consent of the mother. The samples were collected at dawn and were processed during the next 12 hours post partum. Mononuclear (MN) cells were obtained by depositing the blood on Ficoll-Hypaque (1.077 g/ml; Pharmacia Biotech, Uppsala, Sweden), and centrifuging them at 400g for 30 minutes. Then the middle layer between the Ficoll and the plasma was collected, and was washed three times, and was then resuspended in Iscove's modified Dulbecco's medium (IMDM; Gibco-BRL, Grand Island, NY). To purify the CD34⁺ cells, the MN fraction was submitted to immunomagnetic separation using the VarioMACS CD34 stem cell isolating kit (Miltenyi Biotech, Auburn, CA), following the manufacturer's recommendations.

The CD34⁺ cells were cryopreserved in liquid nitrogen, with 10% dimethylsulphoxide (DMSO) and 20% fetal bovine serum (FBS) until they were used.

### Example 10. Transduction, culture and transplantation in NOD/SCID mice.

After thawing, the CD34⁺ cells were washed in IMDM supplemented with 10% FBS and antibiotics (100 U/ml penicillin and 50 µg/ml streptomycin). Then the cells were resuspended in order to be pre-stimulated for 2 hours with StemSpan serum-free medium (Stem Cell Technologies Inc., Vancouver, BC, Canada) supplemented with antibiotics (100 U/ml penicillin and 50 µg/ml streptomycin), 300 ng/ml of stem cell factor (SCF, kindly supplied by Amgen, Thousand Oaks, CA), 100 ng/ml of thrombopoietin (TPO, kindly supplied by Kirin Brewery, Tokyo, Japan) and 100 ng/ml ofprogenipoietin (ProGP, kindly supplied by Monsanto Company, St. Louis, MO).

These cells, positive for CD34, were then transduced at two MOI (3 and 0.3 pfu/cell) with the *Lentivirus* supernatants both with CMV and with HS1 for 24 hours. During this time the cells were centrifuged, 2 and 20 hours after transduction, for 1 hour at 2500 rpm and 37°C. Then the cells were washed, and 2 to 3x10⁶ cells per mouse were transplanted in lethally irradiated NOD/SCID recipient mice. Following transplant, periodically, bone marrow samples from these animals were analysed by flow cytometry to determine the degree of implantation of the human transplant. These bone marrow samples were aspirated from a femur by puncture through the knee joint, in accordance with procedures previously described (Verlinden SF et al., Exp. Hematol. 26:627, 1998).

The data from the examples are presented in the form of the mean ± standard deviation from the mean. The significance of the differences between data sets was determined using the Student *t*-test. Statistical analysis of the data was performed using the Stat graphics Plus program (Maugistics Inc., Rockville, MD, USA).

### REFERENCES

1. Cavazzana-Calvo M, Hacein-Bey S, de Saint Basile G, Gross F, Nusbaum P, Yvon E, Casanova JL, Le Deist F, Fischer A. Correction of SCID-X1 disease phenotype following gamma-c gene transfer by a retroviral vector into CD34+ cells in two children. Blood. 1999;94:367
2. Cavazzana-Calvo M, Hacein-Bey S, de Saint Basile G, Gross F, Yvon E, Nusbaum P, Selz F, Hue C, Certain S, Casanova JL, Bousso P, Deist FL, Fischer A. Gene therapy of human severe combined immunodeficiency (SCID)-X1 disease. Science. 2000:288:669-672.
3. Kustikova O, Fehse B, Modlich U, Yang M, Dullmann J, Kamino K, von NeuhoffN, Schlegelberger B, Li Z, Baum C. Clonal Dominance of Hematopoietic Stem Cells Triggered by Retroviral Gene Marking. Science. 2005;308:1171-1174
4. Li Z, Dullmann J, Schiedlmeier B, Schmidt M, von Kalle C, Meyer J, Forster M, Stocking C, Wahlers A, Frank O, Ostertag W, Kuhlcke K, Eckert HG, Fehse B, Baum C. Murine leukemia induced by retroviral gene marking. Science. 2002;296:497.
5. Gaspar HB, Parsley KL, Howe S, King D, Gilmour KC, Sinclair J, Brouns G, Schmidt M, Von Kalle C, Barington T, Jakobsen MA, Christensen HO, Al Ghonaium A, White HN, Smith JL, Levinsky RJ, Ali RR, Kinnon C, Thrasher AJ. Gene therapy of X-linked severe combined immunodeficiency by use of a pseudotyped gammaretroviral vector. Lancet. 2004;364:2181-2187.
6. Aiuti A, Slavin S, Aker M, Ficara F, Deola S, Mortellaro A, Morecki S, Andolfi G, Tabucchi A, Carlucci F, Marinello E, Cattaneo F, Vai S, Servida P, Miniero R, Roncarolo MG, Bordignon C. Correction of ADA-SCID by stem cell gene therapy combined with. Science. 2002;296:2410-2413.
7. Baum C, Dullmann J, Li Z, Fehse B, Meyer J, Williams DA, von Kalle C. Side effects of retroviral gene transfer into hematopoietic stem cells. Blood. 2003;101:2099-2114.
8. Baum C, von Kalle C, Staal FJ, Li Z, Fehse B, Schmidt M, Weerkamp F, Karlsson S, Wagemaker G, Williams DA. Chance or necessity? Insertional mutagenesis in gene therapy and its consequences. Mol Ther. 2004;9:5-13.
9. Hacein-Bey-Abina S, von Kalle C, Schmidt M, Le Deist F, Wulffraat N, Mclntyre E, Radford I, Villeval JL, Fraser CC, Cavazzana-Calvo M, Fischer A. A serious adverse event after successful gene therapy for X-linked severe combined immunodeficiency. N Engl J Med. 2003;348:255-256.
10. Hacein-Bey-Abina S, Von Kalle C, Schmidt M, McCormack MP, Wulffraat N, Leboulch P, Lim A, Osborne CS, Pawliuk R, Morillon E, Sorensen R, Forster A, Fraser P, Cohen JI, de Saint Basile G, Alexander I, Wintergerst U, Frebourg T, Aurias A, Stoppa-Lyonnet D, Romana S, Radford-Weiss I, Gross F, Valensi F, Delabesse E, Macintyre E, Sigaux F, Soulier J, Leiva LE, Wissler M, Prinz C, Rabbitts TH, Le Deist F, Fischer A, Cavazzana-Calvo M. LMO2-associated clonal T cell proliferation in two patients after gene therapy for SCID-X1. Science. 2003;302:415-419.
11. Schroder AR, Shinn P, Chen H, Berry C, Ecker JR, Bushman F. HIV-1 integration in the human genome favors active genes and local. Cell. 2002;110:521-529.
12. Wu X, Li Y, Crise B, Burgess SM. Transcription start regions in the human genome are favored targets for MLV integration. Science. 2003;300:1749-1751.
13. Mitchell RS, Beitzel BF, Schroder AR, Shinn P, Chen H, Berry CC, Ecker JR, Bushman FD. Retroviral DNA integration: ASLV, HIV, and MLV show distinct target site preferences. PLoS Biol. 2004;2:E234.
14. Recchia A, Bonini C, Magnani Z, Urbinati F, Sartori D, Muraro S, Tagliafico E, Bondanza A, Stanghellini MT, Bernardi M, Pescarollo A, Ciceri F, Bordignon C, Mavilio F. Retroviral vector integration deregulates gene expression but has no consequence on the biology and function of transplanted T cells. Proc Natl Acad Sci U S A. 2006;103:1457-1462.
15. Ogilvy S, Elefanty AG, Visvader J, Bath ML, Harris AW, Adams JM. Transcriptional regulation of vav, a gene expressed throughout the hematopoietic compartment. Blood. 1998;91:419-430.
16. Ogilvy S, Metcalf D, Gibson L, Bath ML, Harris AW, Adams JM. Promoter elements of vav drive transgene expression in vivo throughout the hematopoietic compartment. Blood. 1999;94:1855-1863.
17. Almarza E, Segovia JC, Guenechea G, Gomez SG, Ramirez A, Bueren JA. Regulatory elements of the vav gene drive transgene expression in hematopoietic stem cells from adult mice. Exp Hematol. 2004;32:360-364.
18.Follenzi, A., G. Sabatino, et al. (2002). "Efficient gene delivery and targeted expression to hepatocytes in vivo by improved lentiviral vectors." Hum Gene Ther 13(2): 243-60.
19. VandenDriessche, T., L. Thorrez, et al. (2002). "Lentiviral vectors containing the human immunodeficiency virus type-1 central polypurine tract can efficiently transduce nondividing hepatocytes and antigen-presenting cells in vivo." Blood 100(3): 813-22.
20. Fehse, B., O. S. Kustikova, et al. (2004). "Pois(s)on--it's a question of dose." Gene Ther 11(11): 879-81.
21. Varas, F., A. Bernad, et al. (1996). "Relevance of myeloablative conditioning in the engraftment of limiting numbers of normal and genetically marked lymphohematopoietic stem cells." Bone Marrow Transplant 18(5): 981-9.

## Claims

1. Use of a vav oncogene promoter consisting of the HS1 promoter of SEQ ID NO:1, in the production of virus-based integrative vectors comprising a promoter consisting of the HS1 promoter of SEQ ID NO:1 for use in the preparation of pharmaceutical compositions intended for somatic gene therapy.

2. The use according to claim 1, **characterized in that** said therapy is carried out "*in vivo*" in mammals.

3. The use according to claim 2, wherein the virus-based integrative vector is selected from the group comprising gamma-retroviruses, lentiviruses, foamy viruses and adeno-associated viruses.

4. A virus-based integrative vector **characterized in that** it comprises a promoter consisting of the vav oncogene HS1 promoter of SEQ ID NO:1.

5. Virus-based integrative vector according to claim 4, **characterized in that** it is a gamma-retrovirus.

6. Virus-based integrative vector according to claim 4, **characterized in that** it is a lentivirus.

7. Virus-based integrative vector according to claim 6, which is a lentiviral vector in which the EGFP gene is under the control of the HS1-vav promoter.

8. Virus-based integrative vector according to claim 4, **characterized in that** it is a foamy virus.

9. Virus-based integrative vector according to claim 4, **characterized in that** it is an adeno-associated virus.

10. Use of the vectors according to Claims 4-9 for the preparation of pharmaceutical compositions intended for use in somatic gene therapy.

11. Cells transduced by the vectors according to Claims 4-9, **characterized in that** they express a gene under the control of a promoter consisting of the *vav* oncogene HS1 promoter of SEQ ID NO:1.

12. Use of the cells according to Claim 11 for the preparation of pharmaceutical compositions intended for use in somatic gene therapy.

13. Pharmaceutical compositions comprising vectors according to Claims 4-9 and pharmaceutically acceptable vehicles.

14. Pharmaceutical compositions comprising the cells according to Claim 11 and pharmaceutically acceptable vehicles.

## Patentansprüche

1. Verwendung eines *vav*-Onkogenpromotors bestehend aus dem HS1-Promotor von SEQ ID NO: 1 in der Herstellung von auf einem Virus basierenden integrativen Vektoren umfassend einen Promotor bestehend aus dem HS1-Promotor von SEQ ID NO: 1 zur Verwendung in der Herstellung von Arzneimitteln, welche zur somatischen Gentherapie vorgesehen sind.

2. Verwendung nach Anspruch 1, **dadurch gekennzeichnet, dass** die Therapie "*in vivo"* in Säugern durchgeführt wird.

3. Verwendung nach Anspruch 2, wobei der auf einem Virus basierende integrative Vektor ausgewählt ist aus der Gruppe umfassend gamma-Retroviren, Lentiviren, Foamyviren und adenoassoziierte Viren.

4. Auf einem Virus basierender integrativer Vektor, **dadurch gekennzeichnet, dass** er einen Promotor bestehend aus dem vav-Onkogen-HS1-Promotor von SEQ ID NO: 1 umfasst.

5. Auf einem Virus basierender integrativer Vektor nach Anspruch 4, **dadurch gekennzeichnet, dass** es ein gamma-Retrovirus ist.

6. Auf einem Virus basierender integrativer Vektor nach Anspruch 4, **dadurch gekennzeichnet, dass** es ein Lentivirus ist.

7. Auf einem Virus basierender integrativer Vektor nach Anspruch 6, welcher ein lentiviraler Vektor ist, in welchem das EGFP-Gen unter der Kontrolle des HS1-*vav*-Promotors steht.

8. Auf einem Virus basierender integrativer Vektor nach Anspruch 4, **dadurch gekennzeichnet, dass** es ein Foamyvirus ist.

9. Auf einem Virus basierender integrativer Vektor nach Anspruch 4, **dadurch gekennzeichnet, dass** es ein adenoassoziierter Virus ist.

10. Verwendung der Vektoren nach den Ansprüchen 4 bis 9 zur Herstellung von Arzneimitteln, welche zur somatischen Gentherapie vorgesehen sind.

11. Zellen, welche durch die Vektoren nach den Ansprüchen 4 bis 9 transduziert sind, **dadurch gekennzeichnet, dass** sie ein Gen unter der Kontrolle eines Promotors, welcher aus dem *vav*-Onkogen-HS1-Promotor von SEQ ID NO: 1 besteht, exprimieren.

12. Verwendung der Zellen nach Anspruch 11 zur Herstellung von Arzneimitteln, welche zur somatischen Gentherapie vorgesehen sind.

13. Arzneimittel umfassend Vektoren nach den Ansprüchen 4 bis 9 und pharmazeutisch verträgliche Hilfsstoffe.

14. Arzneimittel umfassend die Zellen nach Anspruch 11 und pharmazeutisch verträgliche Hilfsstoffe.

## Revendications

1. Utilisation d'un promoteur d'oncogène vav constitué par le promoteur HS1 de SEQ ID No.: 1, dans la production de vecteurs d'intégration viraux comprenant un promoteur constitué par le promoteur HS1 de SEQ ID No.: 1 pouvant être utilisés dans la préparation de compositions pharmaceutiques destinées à la thérapie génique somatique.

2. Utilisation selon la revendication 1, **caractérisée en ce que** ladite thérapie est mise en oeuvre "in vivo" chez les mammifères.

3. Utilisation selon la revendication 2, dans laquelle le vecteur d'intégration viral est choisi dans le groupe constitué par les gamma-rétrovirus, les lentivirus, les virus syncytiaux et les virus adéno-associés.

4. Vecteur d'intégration viral **caractérisé en ce qu'**il comprend un promoteur constitué par le promoteur HS1 de l'oncogène vav de SEQ ID No.: 1.

5. Vecteur d'intégration viral selon la revendication 4, **caractérisé en ce que** c'est un gamma-rétrovirus.

6. Vecteur d'intégration viral selon la revendication 4, **caractérisé en ce que** c'est un lentivirus.

7. Vecteur d'intégration viral selon la revendication 6, qui est un vecteur lentiviral dans lequel le gène EGFP est sous le contrôle du promoteur HS1-vav.

8. Vecteur d'intégration viral selon la revendication 4, **caractérisé en ce que** c'est un virus syncytial.

9. Vecteur d'intégration viral selon la revendication 4, **caractérisé en ce que** c'est un virus adéno-associé.

10. Utilisation des vecteurs selon les revendications 4 à 9 pour la préparation de compositions pharmaceutiques destinées à une utilisation en thérapie génique somatique.

11. Cellules transduites par les vecteurs selon les revendications 4 à 9, **caractérisées en ce qu'**elles expriment un gène sous le contrôle d'un promoteur constitué par le promoteur HS1 de l'oncogène vav de SEQ ID No.: 1.

12. Utilisation des cellules selon la revendication 11 pour la préparation de compositions pharmaceutiques destinées à une utilisation en thérapie génique somatique.

13. Compositions pharmaceutiques comprenant des vecteurs selon les revendications 4 à 9 et des véhicules pharmaceutiquement acceptables.

14. Compositions pharmaceutiques comprenant les cellules selon la revendication 11 et des véhicules pharmaceutiquement acceptables.


<110> CIEMAT. Energy, Environment and Technology Research Centre.
<120> Expression vectors comprising the HS1 promoter of the vav1 oncogene and use thereof for the preparation of pharmaceutical compositions intended for somatic gene therapy.
<130> P-100443
<160> 1
<210> SEQ ID NO: 1
   <211> 1195
   <212> DNA
   <213> *Mus musculus*
   <221> vavHS1 promoter.
<400>
   CAATCGTTGG GGCTTGCATG GTGGAAAGAG GACCTACTCC CCCACGTTAA 50
   CCTTTGACCT CCACACATGT ACTGTGCCCC ACACGTACCT TCACACATAC 100
   ACACATACAT ATGTATATAT ATACACACAC ACACATATAT ATATGTAATA 150
   AAAGTAAAAT GGCTTTAAAA GTAGAATATA TTTTCTAATG TTTATTATAG 200
   TTCTATTTGT TGGCAGTTTC CTACATGTTA ACAACCTATC TTCGTCCTAC 250
   CCACCCTCAA CTCCCCTCTG ACTCCGAATG AGAGTGGACT CTCTTTACCT 300
   TATCCCCACT TTCTTGCTCT TTTGTAACCC TTTGCATCCA TTACTGCTGC 350
   CTGTGGGCAT GTTGGCTCCC TCCTGGCTTC GTCTTGTGCT GTCACCATAA 400
   CTGCACGGAG TTCATGAGCG TAGTGGCCAT GGGTATGCCC TGGAGATAGC 450
   ATTTCACATC CCCCGCCCCC GACTCTTACC GACTTTCTGT TCCTTCTCCC 500
   TTGAGCCTCG GGCCGGTGGT GGTATAGATG TCCCATCTAT GGCTGAGCAC 550
   TCAGTAATCA AAAAGTAACA TGTAAGAAGT GATGGAGTGA ATTAGTGTCT 600
   TCTGTCAGGT GGTGGGCACA AGTGCAAAGG CCCTGGGGCA GGATGGAGGT 650
   CAGGGAGTGA AGAACAGAGA GAGGAGAAGA GGCTGGCAGG GGTTGGGGCA 700
   GGGTGAACAG GGCCTTGTGG GGTGCAGGAA AGACATGAGT TTTGGCCCCA 750
   AAGCAAGGTT GAACCAGGAG GGTTATGGGT GGTGGAAGAA TGGTTGAGAC 800
   TTGACTCAGT GTTCAGTGGT CCCTTTTGGC TGCTGCAGGG GCAAAAGGCT 850
   GTGAGAGATC AGGGCCGTAG ACTGGGGACC AGGAGGAGAC AGGAGGTGAC 900
   AGCCTAGGTG CCTACGGCTG GATGAGATAG TGGCACCTTA GTTCAGCAGG 950
   TGCCCAGGTC TGCTCAGTGT AGGTGGAGAA AGACGAGGTC ACACCAGTGA 1000
   GTGGAAGCCG TGAGGGCGTA ACTGCAGATG CTGCTCCCCC TCCGCCCCAC 1050
   AGCTCCTCCC CACCCCCACC CCGGGGCGAC AGTTACAGTC ACAGAAGAGG 1100
   AAGTGGTGTT GTAGTTGTCC CCACTGGGGC AGGCGGCGGC GGTGGTGAAG 1150
   AACGAGGGTG CACGGGGCCT CCCCGAGGGG CCAAGTGAGA GGCCG 1195